# EUROPÄISCHE PATENTANMELDUNG

(11) **EP 4 406 724 A2**
(43) Veröffentlichungstag der Anmeldung: **31.07.2024**
(21) Anmeldenummer: 23214573.0
(22) Anmeldetag: 06.12.2023
(51) Int. Cl.: B29C 49/42, F16K 31/122, A61L 2/20, B29C 49/12, B29C 49/36, B29C 49/46, B29C 49/78, B29L 31/00, F16K 31/126

(54) **BLASFORMMASCHINE MIT ASEPTISCHEN VENTILEN**

(30) Priorität: 25.01.2023 DE 102023101825
(71) Anmelder: Krones AG, 93073 Neutraubling (DE)
(72) Erfinder: Buess, Armin, 93073 Neutraubling (DE); Habenschaden, Nina, 93073 Neutraubling (DE); Senn, Konrad, 93073 Neutraubling (DE); Hoellriegl, Thomas, 93073 Neutraubling (DE)
(74) Vertreter: Bittner, Bernhard

(57) **Zusammenfassung**

Vorrichtung zum Umformen von Kunststoffvorformlingen (10) zu Kunststoffbehältnissen, mit einer Transporteinrichtung (2), welche die Kunststoffvorformlinge (10) entlang eines vorgegebenen Transportpfads transportiert, wobei die Transporteinrichtung (2) einen Transportträger (22) aufweist, an dem eine Vielzahl von Umformungsstationen (20) zum Umformen der Kunststoffvorformlinge (10) angeordnet ist, wobei diese Umformungsstationen jeweils Beaufschlagungseinrichtungen (84) aufweisen, welche die Kunststoffvorformlinge zu ihrer Expansion mit einem fließfähigen und insbesondere gasförmigen Medium beaufschlagen und wobei die Vorrichtung (1) wenigstens ein erstes Druckluftreservoir (32) aufweist, welches dazu geeignet und bestimmt ist, Druckluft unter einem ersten vorgegebenen Druck zu speichern und jede Umformungsstation (20) eine Ventilanordnung (4) mit einer Vielzahl von Ventileinrichtungen (50) aufweist, um die Kunststoffvorformlinge mit wenigstens zwei Drücken zu beaufschlagen, dadurch gekennzeichnet, dass wenigstens eine dieser Ventileinrichtungen (50) eine durch ein Sterilisationsmedium sterilisierbare pneumatisch gesteuerte Ventileinrichtung oder eine durch ein Sterilisationsmedium sterilisierbare magnetisch und/oder durch Magnetkraft betätigbare Ventileinrichtung ist.

## Beschreibung

Die vorliegende Erfindung bezieht sich auf eine Vorrichtung und ein Verfahren zum Umformen von Kunststoffvorformlingen zu Kunststoffbehältnissen. Derartige Vorrichtungen und Verfahren sind aus dem Stand der Technik seit langem bekannt. Dabei werden üblicherweise erwärmte Kunststoffvorformlinge durch Beaufschlagung mit Druckluft zu Kunststoffbehältnissen und insbesondere zu Kunststoffflaschen umgeformt. Zu diesem Zweck werden die Kunststoffvorformlinge mit wenigstens zwei, neuerdings mit mehreren unterschiedlichen Druckniveaus beaufschlagt. Üblicherweise ist dabei eine Vielzahl von Umformungsstationen an einen drehbaren Träger, dem sogenannten Blasrad angeordnet. Die einzelnen Umformungsstationen weisen dabei jeweils Ventilblöcke bzw. Ventilanordnungen auf, die eine Vielzahl von Ventilen aufweisen. Bei diesen Ventilen handelt es sich teilweise um pneumatisch gesteuerte Ventile.

In jüngerer Zeit sind auch Blasformmaschinen oder Umformungseinrichtungen bekannt geworden, welche die Behältnisse unter aseptischen oder sterilen Bedingungen umformen. Dies bedeutet, dass im Stand der Technik aber bevorzugt auch im Falle der Erfindung die Blasformmaschine in einem von einem Produktionsbetrieb unterschiedlichen Sterilisationsbetrieb mit einem Sterilisationsmedium, wie zum Beispiel Wasserstoffperoxid, sterilisiert wird. Nach dem Sterilisationsbetrieb werden die Behältnisse unter sterilen Bedingungen mit Sterilluft, insbesondere mit gefilterter Luft, umgeformt. Während dem Sterilisationsbetrieb werden auch die Ventilblöcke bzw. die Ventile mit Sterilisationsmedium sterilisiert.

Während dem Arbeitsbetrieb, ist die jeweilige Steuerluft zum Ansteuern der Ventile jedoch üblicherweise nicht steril.

Bei Ventilanordnungen oder Ventilblöcken in einem Sterilraum oder allgemein einem aseptischen Raum sollte gewährleistet sein, dass die zu schaltende aseptische Blasluft nicht mit der Steuerluft der Ventile verunreinigt wird. Aus diesem Grund wird im Stand der Technik teilweise eine hermetische Abdichtung der von der Blasluft durchströmten Bereiche gegenüber der Steuerluft gefordert. Zu diesem Zweck kann beispielsweise das Hubventil mittels eines Faltenbalgs oder einer Membran hermetisch zum Ventilgehäuse abgedichtet werden. Aus dem Stand der Technik bekannte Blasblöcke oder Ventilanordnungen sind nicht sterilisierbar, da zum einen die Sterilgrenzen nicht definiert sind und zum anderen die Materialien nicht passend ausgelegt sind. Insbesondere sind im Stand der Technik Ventileinrichtung nicht sterilisierbar, wenn sie keine trennende Membran oder einen trennenden Balg aufweisen.

In der Praxis kann es vorkommen, dass derartige Faltenbälge reißen. Daneben haben derartige Ventile den Nachteil, dass sie einen größeren Bauraum im Vergleich zu einem balglosen Ventil benötigen. Daneben sind üblicherweise die Geometrien derartiger Bälge oder die Werkstoffe an ihren Belastungsgrenzen. Weiterhin wird das Material des Balges nach der Sterilisation durch eine hohe Temperatur weich, sodass bei einem sofortigen Produktionsstart hierdurch Defekte an dem Balg entstehen.

Daneben benötigt ein derartiges Ventil zwei Piloten und/oder Steuerdrücke zum Öffnen und Schließen, was zu höheren Kosten und einem höheren Platzbedarf führt.

Der vorliegenden Erfindung liegt daher die Aufgabe zugrunde, eine Blasformmaschine zur Verfügung zu stellen, welche insbesondere für Sterilraumanwendungen vielseitiger einsetzbar ist, welche jedoch weiterhin auch den eigentlichen Blasvorgang, insbesondere bei der Beaufschlagung mit unterschiedlichen Drücken vereinfacht. Dies wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Umformen von Kunststoffvorformlingen zu Kunststoffbehältnissen weist eine Transporteinrichtung auf, welche die Kunststoffvorformlinge entlang eines vorgegebenen Transportpfads transportiert. Dabei weist die Transporteinrichtung einen (bewegbaren und insbesondere drehbaren) Transportträger auf, an dem eine Vielzahl von Umformungsstationen zum Umformen der Vorformlinge zu Kunststoffbehältnissen angeordnet ist, wobei diese Umformungsstationen jeweils Beaufschlagungseinrichtungen aufweisen, welche die Kunststoffvorformlinge zu ihrer Expansion mit einem fließfähigen und insbesondere gasförmigen Medium beaufschlagen.

Daneben weist die Vorrichtung wenigstens ein erstes Druckluftreservoir auf, welches dazu geeignet und bestimmt ist, Druckluft unter einem ersten vorgegebenen Druck zu speichern und/oder bereitzustellen und jede Umformungsstation weist eine Ventilanordnung, mit einer Vielzahl von Ventileinrichtungen auf, um die Kunststoffvorformlinge mit wenigstens zwei (unterschiedlichen) Drücken zu beaufschlagen.

Erfindungsgemäß ist wenigstens eine dieser Ventileinrichtungen eine durch ein Sterilisationsmedium sterilisierbare pneumatisch gesteuerte Ventileinrichtung oder eine durch ein Sterilisationsmedium sterilisierbare magnetisch und/oder durch Magnetkraft betätigbare Ventileinrichtung. Die Sterilisation erfolgt in einem von einem Produktionsbetrieb unterschiedlichen Sterilisationsbetrieb.

Unter einer pneumatisch gesteuerten Ventileinrichtung wird insbesondere verstanden, dass Steuerluft oder dergleichen vorgesehen ist, um dieses Ventil von einer geschlossenen Stellung in eine geöffnete Stellung und/oder einer geöffneten Stellung in eine geschlossene Stellung zu schalten. In der geöffneten Stellung erfolgt bevorzugt eine Druckbeaufschlagung der Kunststoffvorformlinge mit einem Arbeitsdruck.

Unter einer magnetisch und/oder durch Magnetkraft betätigbaren Ventileinrichtung wird verstanden, dass zumindest eine dieser Ventilbewegungen durch eine Magnetkraft erreicht oder durchgeführt wird. Dies wird unter Bezugnahme auf die Figuren genauer erläutert.

Es wäre auch denkbar, dass die Ventileinrichtung sowohl durch eine Steuerluft betätigt wird als auch eine Magnetkraft die eigentliche Bewegung eines Ventilkolbens bewirkt, bzw. eine entsprechende Kraftübertragung durch Magnetkräfte bewirkt wird.

Bei einer bevorzugten Ausführungsform weist die Ventileinrichtung einen beweglichen und insbesondere linearbeweglichen Ventilkolben auf, wobei in einer ersten Stellung dieses Ventilkolbens die Ventileinrichtung geschlossen ist und damit kein gasförmiges Medium durch die Ventileinrichtung zu der Beaufschlagungseinrichtung gelangt. Bevorzugt ist in einer zweiten Stellung dieses Ventilkolbens die Ventileinrichtung geöffnet und das gasförmige Medium bzw. die Blasluft gelangt zu der Beaufschlagungseinrichtung.

Bei dem fließfähigen Sterilisationsmedium kann es sich insbesondere um Wasserstoffperoxid oder Peressigsäure handeln.

Bevorzugt weisen die Beaufschlagungseinrichtungen jeweils Blasdüsen auf oder sind Blasdüsen. Diese können dabei an eine Mündung der zu expandierenden Kunststoffvorformlinge oder auch am Bereich einer Blasform gelegt werden, um die Kunststoffvorformlinge so mit den einzelnen Drücken zu ihrer Expansion zu beaufschlagen.

Weiterhin weisen die Umformungsstationen bevorzugt jeweils Blasformen auf, innerhalb derer die Kunststoffvorformlinge zu den Kunststoffbehältnissen expandiert bzw. geblasen werden.

Besonders bevorzugt sind diese Blasformen dabei wenigstens zweiteilig und bevorzugt wenigstens dreiteilig aufgebaut und weisen beispielsweise zwei Seitenteile und ein Bodenteil auf.

Besonders bevorzugt ist das Druckluftreservoir, welches das fließfähige Medium zur Umformung der Behälterbereitstellt mit den einzelnen Ventilanordnungen bzw. Ventilblöcken und genauer mit den einzelnen Ventileinrichtungen in Strömungsverbindung oder kann in eine solche gebracht werden. Dabei kann dies derart erfolgen, dass der entsprechende Arbeitsdruck stets an der Ventileinrichtung anliegt.

Bei dem Druckreservoir kann es sich um einen Behälter handeln. Daneben kann das Reservoir auch aus Schläuchen, Kanälen oder Rohren gebildet sein. Bei dem Begriff des Reservoirs steht nicht primär die Speicherfähigkeit sondern die Gemeinsamkeit (für alle Umformungsstationen) im Vordergrund. So wäre es möglich, auf Ringkessel oder Ringkanäle zu verzichten und direkt mit Schläuchen zur Umformungsstationen zu gehen. Dabei verbinden die Schläuche mit T stücken oder zentral Verteilern mindestens 2 Umformungsstationen miteinander, so dass diese drucktechnisch "kommunizieren" können.

Bei einer bevorzugten Ausführungsform handelt es sich bei den Reservoirs um Ringkanäle. Besonders bevorzugt ist das wenigstens eine Reservoir und bevorzugt die mehreren Reservoirs an dem drehbaren Träger, im Folgenden auch als Blasrad bezeichnet, angeordnet.

Bei einer weiteren vorteilhaften Ausführungsform sind alle Teile der Ventileinrichtung, welche mit dem Sterilisationsmedium in Kontakt kommen, aus Materialen, welche gegen Sterilisationsmedien und Sterilisationstemperatur beständig sind und welche eine ausreichende Oberflächenbeschaffenheit aufweisen, um eine Sterilisation zu erlauben. Daneben handelt es sich bevorzugt um Materialien welche resistent gegen Sterilisationsmedium sind.

Bei einer weiteren bevorzugten Ausführungsform weist die Vorrichtung eine Sterilisationseinrichtung auf, welche dazu geeignet ist, ein Ventil mit dem Sterilisationsmedium zu beaufschlagen. Insbesondere können dabei diejenigen Strömungswege oder diejenigen Räume der Ventileinrichtung mit dem Sterilisationsmedium beaufschlagt werden, welche in einem Arbeitsbetrieb auch das Arbeitsfluid, insbesondere die Blasluft fördern.

Besonders bevorzugt weist die Ventileinrichtung einen Ventilkolben auf, der bevorzugt in einer vorgegebenen linearen Richtung beweglich ist. Dieser bewegliche Kolben dient damit zum Steuern der Blasluft (im Rahmen der vorliegenden Anmeldung teilweise auch als Arbeitsluft bezeichnet), und kann in einer Stellung das Ventil schließen und in einer anderen Stellung das Ventil öffnen.

Besonders bevorzugt ist dieser Ventilkolben in einem aseptischen Raum der Vorrichtung angeordnet.

Bei einer bevorzugten Ausführungsform handelt es sich bei der Ventileinrichtung um ein aseptisches bzw. pneumatisch und/oder magnetisch gesteuertes Ventil und wie unten genau beschrieben wird, um ein balgloses Ventil.

Besonders bevorzugt ist sowohl ein Steuerraum dieser Ventileinrichtung, in welchem die Steuerluft zum Schalten des Ventils geführt wird, als auch ein Arbeitsraum dieser Ventileinrichtung, in welchem die Blasluft zum Expandieren der Kunststoffvorformlinge geführt und/oder wenigstens zeitweise gespeichert wird, vollständig sterilisierbar.

Bei aus dem Stand der Technik bekannten Ventilen sind keine Ventile bekannt, bei welchen der Ventilkolben komplett sterilisierbar ist.

Bei einer bevorzugten Ausführungsform weist die Ventileinrichtung ein Ventil, insbesondere ein Wegeventil und insbesondere ein aseptisches Wegeventil auf. Dieses Ventil steuert wiederum ein Kolbenventil und/oder den oben erwähnten Ventilkolben an (insbesondere um die Blasluft zu sperren oder sie zu der Beaufschlagungseinrichtung gelangen zu lassen).

Dabei ist es möglich, dass dieses Wegeventil mit einem Balg oder einer Membran versehen ist, sodass auf diese Weise eine Trennung zwischen einer aseptischen Steuerluft und einer nicht aseptischen Steuerluft stattfinden kann.

Bei einer bevorzugten Ausführungsform ist die Ventileinrichtung balglos ausgeführt. Im Stand der Technik sind teilweise Faltenbälge vorgesehen, welche die Ventileinrichtung in einen aseptischen und nicht aseptischen Bereich trennen. Diese Bälge haben oft den Nachteil, dass sie von Sterilisationsmitteln angegriffen werden und auf diese Weise beschädigt werden. Außerdem halten derartiger Bälge oftmals Erwärmungen nicht stand. Dies bedeutet, dass in den Fällen, in denen man sehr schnell nach der Sterilisation des Ventils in einen Arbeitsbetrieb übergeht, das Material noch zu weich ist und es so zu Störungen oder Beschädigungen des Balgs führen kann.

Bei einer bevorzugten Ausführungsform weist die Ventileinrichtung einen Arbeitsraum auf, welcher dazu geeignet und bestimmt ist, wenigstens zeitweise das zur Expansion der Kunststoffvorformlinge verwendete gasförmige Medium aufzunehmen. Weiterhin weist das Ventil einen Steuerraum zum Aufnehmen des zum Steuern der Ventileinrichtung verwendeten gasförmigen Mediums, insbesondere Luft, auf. Bevorzugt ist dabei sowohl der Arbeitsraum als auch der Steuerraum von einem Sterilisationsmedium sterilisierbar.

Besonders bevorzugt sind der Steuerraum und der Arbeitsraum fluidtechnisch vollständig voneinander getrennt. Im Stand der Technik wird zum Steuern des Ventils nicht-aseptische Luft verwendet. Durch diese Ausgestaltung wird verhindert, dass diese Luft die eigentliche Arbeitsluft verunreinigen kann und auf diese Weise wird verhindert, dass letztlich verunreinigte Arbeitsluft in die Behältnisse gelangt.

Bevorzugt wird sterile Luft auch zum Steuern des Ventils verwendet wird. Somit besteht auch keine Gefahr, dass die Arbeitsluft bzw. Blasluft verunreinigt wird.

Bei einer weiteren vorteilhaften Ausführungsform weist die Vorrichtung einen Reinraum auf, innerhalb dessen die umzuformenden Kunststoffvorformlinge transportiert werden. Insbesondere sind die Kunststoffvorformlinge während ihrer Expansion durch diesen Reinraum transportierbar. Bevorzugt sind auch die Umformungsstationen durch diesen Reinraum transportierbar.

Unter einem Reinraum wird ein Raumbereich bzw. ein Raum verstanden, innerhalb dessen die Luft einen höheren Reinheitsgrad bzw. eine geringere Anzahl an Keimen aufweist als in einer Umgebung. Dabei können unterschiedliche Reinheitsgrade vorgesehen sein, beispielsweise ein Reinheitsgrad, der gegenüber der Umgebung nur 1/10 oder auch nur 1/100 der Keimkonzentration aufweist. Bevorzugt ist dieser Reinraum gegenüber einer (unsterilen) Umgebung abgegrenzt.

Besonders bevorzugt werden die Kunststoffvorformlinge während ihrer Beaufschlagung mit dem gasförmigen Medium zu ihrer Expansion durch diesen Reinraum transportiert.

Bevorzugt weist der Reinraum wenigstens zwei diesen Reinraum begrenzende Wandungen auf, die bevorzugt bezüglich einander beweglich sind.

Besonders bevorzugt weist die Vorrichtung eine Dichtungseinrichtung auf, welche den Reinraum gegenüber einer Umgebung abdichtet. Hierbei kann es sich beispielsweise um eine hydraulische Dichtung handeln. Besonders bevorzugt weist die Vorrichtung zwei Dichtungseinrichtungen, insbesondere zwei Wasserschlösser auf, welche dazu geeignet und bestimmt sind, den Reinraum gegenüber der Umgebung abzudichten.

Besonders bevorzugt befindet sich der oben erwähnte Hauptkolben der Ventileinrichtung, der die Blasluft steuert stets innerhalb eines Raums der (insbesondere stets) mit aseptischer Luft umspült werden kann.

Die Ventileinrichtung setzt sich dabei bevorzugt aus einem Steuerventil und einem Hauptventil zusammen und/oder weist diese Elemente auf. Das Steuerventil steuert das Hauptventil mit dem Ventilkolben an, um das Behältnis mit der Blasluft zu beaufschlagen.

Bei einer weiteren vorteilhaften Ausführungsform handelt es sich bei dem Steuerventil um ein 3/2 Wegeventil oder ein 5/2 Wegeventil. Wie unten genauer beschrieben wird, weist ein derartiges 5/2 Wegeventil einen geringeren Hub auf bzw. benötigt einen geringeren Hub. In diesem Fall lässt sich auch ein Balg oder eine Membran leichter realisieren, da diese eine geringere Fläche einnimmt. Auch ein Steuerdruck ist hier geringer.

Bei der Verwendung eines 3/2 Wegeventil wäre es möglich, dass ein Balg oder insbesondere eine vorgesehene Membran eine Trennstelle zwischen der sterile Steuerluft und einer nicht sterilen Pilotluft ist, welche die Ventilbewegung und/oder die Bewegung des oben erwähnten Ventilkolbens realisiert. Mit dieser sterilen Steuerluft wird bevorzugt der Ventilkolben angesteuert, der die Blasluft freigibt.

Bei einer weiteren bevorzugten Ausführungsform wird der Ventilkolben 54 der Ventileinrichtung über eine Luftfeder in die geschlossene Stellung gedrückt. Die Rückstellung kann auch durch eine weiteres 3/2 Wegeventil erfolgen.

Bevorzugt handelt es sich bei der Rückstelleinrichtung um eine Federeinrichtung, insbesondere eine Luftfedereinrichtung. Daneben kann es sich bei der Rückstelleinrichtung auch um eine weitere Ventileinrichtung handeln.

Besonders bevorzugt ist dabei die Vorrichtung derart ausgeführt, dass ein Druck der Blasluft welche zum Expandieren der Behältnisse verwendet wird, stets größer ist als ein Druck der Steuerluft, welche zum Betätigen der Ventileinrichtung dient.

Besonders bevorzugt ist ein Druck der Arbeitsluft größer als 5 bar, bevorzugt größer als 7 bar und bevorzugt größer als 10 bar. Besonders bevorzugt ist ein Druck der Arbeitsluft geringer als 60 bar, bevorzugt geringer als 50 bar, bevorzugt geringer als 40 bar und bevorzugt geringer als 30 bar.

Der Druck der Steuerluft ist besonders bevorzugt größer als 1 bar, bevorzugt größer als 2 bar, bevorzugt größer als 3 bar. Weiterhin ist der Druck der Steuerluft geringer als 15 bar, bevorzugt geringer als 12 bar und besonders bevorzugt geringer als 10 bar. Durch die Ausgestaltung dieser Druckverhältnisse wird erreicht, dass auch bei einer Beschädigung der Membran oder des Balgs kein Eintritt der nicht-aseptischen Steuerluft zu der aseptischen Arbeitsluft auftritt.

Durch die Erfindung wird erreicht, dass eine Bewegung des Hauptkolbens der Ventileinrichtung komplett in einem aseptischen Raum erfolgt. Damit besteht keine Gefahr der Verunreinigung auch nicht im Falle einer Undichtigkeit. Bei einer Undichtigkeit der Membran im Steuerventil kann durch die Druckverhältnisse keine unsterile Luft in die sterile Steuerluft gelangen, da der Druck auf der aseptischen Steuerseite, beispielsweise einer Membran, höher ist als auf der unsterilen Seite. Eine Schaltbarkeit kann gleichwohl über ein unterschiedliches Flächenverhältnis gegeben sein. Besonders bevorzugt ist die Fläche, auf der aseptischen Seite kleiner (als auf der nicht aseptischen Seite) und auf diese Weise wird ein höherer Druck ermöglicht.

Bei einer weiteren vorteilhaften Ausführungsform weist die Vorrichtung ein zweites Druckluftreservoir auf, welches dazu geeignet und bestimmt ist, Druckluft unter einem zweiten vorgebbaren Druck zu bereitzustellen, wobei dieser zweite Druck höher ist als der erste Druck. So kann es sich bei dem zweiten Druck beispielsweise um einen Fertigblasdruck handeln, mit dem das Behältnis bevorzugt endgültig ausgeformt und dessen endgültige Gestalt festgelegt wird.

Bei einer weiteren vorteilhaften Ausführungsform weist die Vorrichtung ein drittes Druckluftreservoirs auf, welches dazu geeignet und bestimmt ist, Druckluft unter einem dritten vorgegebenen Druck speichern, wobei dieser dritte Druck bevorzugt größer ist als der erste Druck und bevorzugt geringer als der zweite Druck.

Bei diesem Druck kann es sich insbesondere um den sogenannten Zwischenblasdruck handeln, der bei neueren Verfahren zum Herstellen von Kunststoffbehältnissen verwendet wird. Besonders bevorzugt ist auch das zweite Druckluftreservoir und das dritte Druckluftreservoir über Ventileinrichtungen in Strömungsverbindung mit der Beaufschlagungseinrichtung und letztlich dem zu expandierenden Kunststoffvorformling bringbar.

Besonders bevorzugt handelt es sich auch bei den Ventileinrichtungen, die die Beaufschlagung des Behältnisses mit dem zweiten Druck und dem dritten Druck ermöglichen, um oben beschriebene Ventile, die besonders bevorzugt vollständig sterilisierbar sind.

Besonders bevorzugt handelt es sich auch bei dem zweiten Druckluftreservoir und/oder dem dritten Druckluftreservoirs um einen Ringkanal, der an dem Blasrad angeordnet ist.

Bei der weiteren vorteilhaften Ausführungsform weisen die Umformungsstationen jeweils stangenartige Körper, insbesondere sogenannte Reckstangen auf, welche in die Kunststoffvorformlinge einführbar sind, um diese in deren Längsrichtung zu dehnen.

Bei einer weiteren vorteilhaften Ausführungsform weist die Ventileinrichtung einen magnetischen Aktuator auf. Bevorzugt ist dieser magnetische Aktuator der Ventileinrichtung durch magnetkraftdurchlässige Wandungen von einem Kolben bzw. einem Ventilkolben der Ventileinrichtung getrennt. Auf diese Weise wird eine magnetische Betätigung der Ventileinrichtung vorgeschlagen, wobei bevorzugt durch die besagte Wandung auch eine Sterilraumgrenze hergestellt wird.

Bei einer bevorzugten Ausführungsform weist die Ventileinrichtung eine Permanentmagnetanordnung auf und insbesondere eine bewegbare Permanentmagnetanordnung. Diese Permanentmagnetanordnung kann durch eine Bewegung auch eine Bewegung des Ventilkolbens hervorrufen.

Besonders bevorzugt ist diese Permanentmagnetanordnung bewegbar und insbesondere linear bewegbar.

Bei einer bevorzugten Ausführungsform weist die Ventileinrichtung einen beweglich und insbesondere schwimmend gelagerten beweglichen Kolben auf. Dieser kann über Magnetkraft mit einem Aktuator verbunden sein. Besonders bevorzugt ist der Aktuator, wie erwähnt, über eine magnetkraftdurchlässige aber aseptisch dichte Grenzschicht, d. h. die oben erwähnte Wandung von dem Kolben und dem Luftraum getrennt. Bei dieser Wandung kann es sich um ein Kunststoffgehäuse handeln, welches insbesondere aber nicht ausschließlich aus Teflon hergestellt sein kann.

Die trennende Wandung bzw. das trennende Gehäuse ist besonders bevorzugt thermisch und chemisch stabil gegenüber Reinigungsmitteln und auch hohen Temperaturen. So kann beispielsweise eine Sterilisation mit H₂O Dampf in einer bestimmten Konzentration über 60° oder über 80° und besonders bevorzugt über 100 °C erfolgen.

Bei einer weiteren bevorzugten Ausführungsform ist die Aktuatoreinrichtung direkt magnetisch. So ist es vorstellbar, dass eine stromdurchflossene Spulenanordnung (direkt oder mit einer Blechung, die die Magnetkraft der Spule richtet) ein Magnetfeld erzeugt, welches einen Kolben der Ventileinrichtung bewegt.

Weiterhin ist es denkbar, dass die Kolbeneinrichtung selbst keinen Permanentmagneten hat, sondern ähnlich wie bei einem asynchronen Linearmotor eine äußere Spule einen Strom in der Kolbeneinrichtung anregt und diese Gegeninduktion und den daraus entstehende Magnetfeld als Aktuatorkraft wirkt.

Bei einer weiteren bevorzugten Ausführungsform ist es auch möglich, die permanentmagnetischen, elektromagnetischen und induktiv induzierbaren magnetischen Kräfte zu kombinieren und/oder mit weiteren Kräften, wie beispielsweise einer mechanischen Feder zu kombinieren.

Bei einer bevorzugten Ausführungsform werden Magneten oberhalb eines (Ventil)Kolbens angesteuert, was bedeutet, dass die Hubeinheit nach unten gedrückt wird und dichtet, beispielsweise auf einer Weichdichtung. Daneben ist es auch möglich, dass Magnete unterhalb angesteuert werden und folglich eine Hubeinheit bzw. ein Kolben nach oben gedrückt wird.

Bei einer weiteren bevorzugten Ausführungsform weist die Ventileinrichtung einen Dichtsitz für einen Ventilkörper auf. Besonders bevorzugt ist dieser Dichtsitz aus einem weichen Material hergestellt. Dies bedeutet, dass anders als bei üblichen Blaskolbenstrukturen welche beispielsweise einen metallischen Dichtsitz haben, auf den ein Kunststoffkolben direkt abdichtet, hier beispielsweise ein Metall wie Aluminium und insbesondere oberflächenbehandeltes Aluminium oder auch Edelstahl oder Titan oder aber ein Kunststoffkolben verwendet wird.

Dieser Kunststoffkolben kann dabei aus einem Material bestehen, welches aus einer Gruppe von Materialien ausgewählt ist, welche PEK, PEI, POM oder PTFE enthält. Dieser Kolben kann dabei auf einem weich dichtendem thermodynamischen Dichtsitz abdichten.

Auf diese Weise kann die notwendige Kraft zum Dichten verringert werden.

Bei einer weiteren bevorzugten Ausführungsform ist der Kolben der Ventileinrichtung (bzw. der Ventilkolben) teilweise oder vollständig druckkompensiert. Dies bedeutet, dass keine oder nur eine sehr kleine resultierende Kraft auf den Kolben aufgrund der anliegenden Drücke wirkt.

Bei einer weiteren vorteilhaften Ausführungsform ist eine Führungseinrichtung des (Ventil)Kolbens vorgesehen. Diese ist bevorzugt derart angeordnet, dass ein Sterilisationsmedium den Kolben überall umschließend erreichen kann. Hiervon ausgenommen kann eine entsprechende Dichtung sein, da diese Stelle dann durch das Bewegen während der Sterilisierens ebenfalls sterilisiert werden kann.

Besonders bevorzugt weist die Ventileinrichtung wenigstens eine Sensoreinrichtung auf. So kann es sich beispielsweise bei der Sensoreinrichtung um eine Druckmesseinrichtung handeln, welche einen Druck der Arbeitsluft und/oder der Steuerluft innerhalb der Ventileinrichtung bestimmt. Auf diese Weise kann sichergestellt werden, dass der Druck der Arbeitsluft stets höher ist als der Druck der Steuerluft.

Bei einer weiteren bevorzugten Ausführungsform können Sensoreinrichtungen an oder in der Ventileinrichtung vorgesehen sein, welche beispielsweise einen Druck oder eine Temperatur und/oder eine Konzentration des Sterilisationsmittels ermitteln. Auf diese Weise ist es möglich, die Sterilisation der Ventileinrichtung zu protokollieren und/oder zu überprüfen, ob eine ordnungsgemäße Sterilisation stattgefunden hat.

Bei einer weiteren bevorzugten Ausführungsform weist die Ventileinrichtung ein Steuerventil und ein Hauptventil auf.

Bevorzugt verläuft dabei eine Sterilgrenze in dem Steuerventil oder ist in dem Steuerventil angeordnet.

Bei einer weiteren bevorzugten Ausführungsform befindet sich ein Hauptkolben der Ventileinrichtung, der die Blasluft steuert stets innerhalb eines Raums, der mit aseptischer Luft umspült werden kann.

Bei einer weiteren bevorzugten Ausführungsform ist dem Steuerventil ein Pilotventil vorgeschaltet, wobei bevorzugt alle Ventileinrichtungen aseptisch ausgeführt sind.

Bei einer weiteren bevorzugten Ausführungsform ist die Vorrichtung sowohl in einem Arbeitsbetrieb betreibbar ist, in welchem die Kunststoffvorformlinge umgeformt werden als auch in einem Sterilisationsbetrieb, in welchem die Vorrichtung und/oder die Ventileinrichtung(en) sterilisiert wird/werden.

Die vorliegende Erfindung ist weiterhin auf ein Verfahren zum Umformen von Kunststoffvorformlingen zu Kunststoffbehältnissen gerichtet, wobei eine Transporteinrichtung die Kunststoffvorformlinge entlang eines vorgegebenen Transportpfads transportiert und wobei die Transporteinrichtung einen beweglichen und insbesondere einen drehbaren Transportträger aufweist, an dem eine Vielzahl von Umformungsstationen zum Umformen der Kunststoffvorformlinge angeordnet ist.

Dabei weisen diese Umformungsstationen jeweils Beaufschlagungseinrichtungen auf, welche die Kunststoffvorformlinge zu ihrer Expansion mit einem fließfähigen und insbesondere gasförmigen Medium beaufschlagen. Weiterhin weist die Vorrichtung wenigstens ein erstes Druckluftreservoir auf, welches Druckluft unter einem ersten vorgegebenen Druck speichert und/oder bereitstellt und jede Umformungsstation weist eine Ventilanordnung mit einer Vielzahl von Ventileinrichtungen auf, um die Kunststoffvorformlinge mit wenigstens zwei unterschiedlichen Drücken zu beaufschlagen.

Erfindungsgemäß ist wenigstens eine dieser Ventileinrichtungen eine durch ein Sterilisationsmedium sterilisierbare und pneumatisch gesteuerte Ventileinrichtung oder eine durch eine Sterilisationsmedium steuerbare magnetisch gesteuerte Ventileinrichtung.

Bei einem weiteren bevorzugten Verfahren herrscht in einem Arbeitsraum der Ventileinrichtung, der zur Aufnahme des die Kunststoffvorformlinge beaufschlagenden gasförmigen Mediums dient, während eines Arbeitsbetriebs ein höherer Druck als in einem Steuerraum, der zu Aufnahme eines Steuerfluids dient, welches einen Ventilkolben der Ventileinrichtung bewegt.

Bei einem weiteren bevorzugten Verfahren werden in wenigstens einem Betriebsmodus und insbesondere während eines Sterilisations Betriebs Parameter dieses Betriebs überwacht. Dabei ist es möglich, dass mit einer Sterilisationsmedium - Ventilschaltung diese Parameter überwacht werden. So können Ventilstellungen und/oder Durchflüsse und/oder Druck und/oder Temperaturen und/oder Konzentrationen überwacht werden.

Auf diese Weise kann eine Sterilisation des gesamten Systems sichergestellt werden.

Bevorzugt werden für diese Vorgänge charakteristische Daten gespeichert, d.h. insbesondere in einer Datenbank gespeichert und abgelegt.

Weitere Vorteile und Ausführungsformen ergeben sich aus den beigefügten Zeichnungen. Darin zeigen:
- Fig. 1: eine schematische Darstellung einer Vorrichtung zum Umformen von Kunststoffvorformlinge zu Kunststoffbehältnissen;
- Fig. 2, 3: zwei Darstellungen einer Vorrichtung mit einem 3/2 Wegeventil und einer Luftfeder;
- Fig. 4a,b: zwei weitere Darstellungen einer erfindungsgemäßen Ventileinrichtung;
- Fig. 5a,b: zwei Darstellungen einer Vorrichtung mit zwei 3/2 Wegeventilen; und
- Fig. 6a,b: zwei Darstellungen einer magnetisch betätigten Ventileinrichtung.

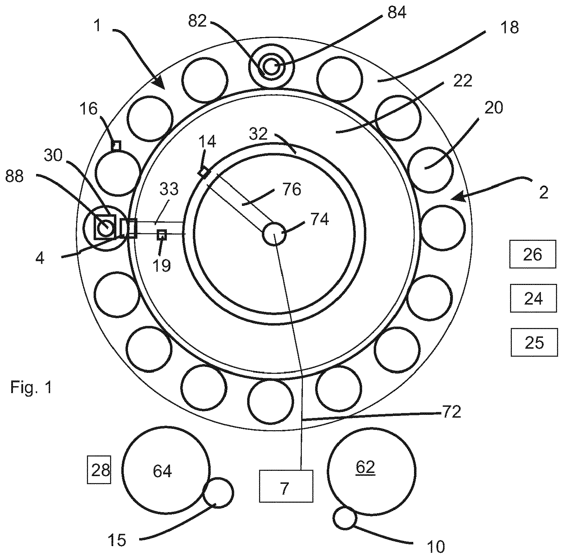

Fig. 1 zeigt eine Vorrichtung 1 zum Umformen von Kunststoffvorformlingen 10 zu Kunststoffbehältnissen 15. Diese Vorrichtung weist eine Transporteinrichtung 2 zum Transportieren der Kunststoffvorformlinge mit einen drehbaren Träger 22 auf, an dem eine Vielzahl von Umformungsstationen 20 angeordnet ist. Diese einzelnen Umformungsstationen weisen jeweils Blasformeinrichtungen 82 auf, die in ihrem Inneren einen Hohlraum zum Expandieren der Kunststoffvorformlinge ausbilden.

Das Bezugszeichen 84 kennzeichnet eine Beaufschlagungseinrichtung, welche zum Expandieren der Kunststoffvorformlinge 10 dient. Hierbei kann es sich beispielsweise um eine Blasdüse handeln, welche an eine Mündung der Kunststoffvorformlinge 10 anlegbar ist, um diese so zu expandieren. Daneben wäre es auch denkbar, dass die Blasdüse an der Blasformeinrichtung abdichtet. Bevorzugt ist diese Beaufschlagungseinrichtung in einer Längsrichtung und bevorzugt ausschließlich in einer Längsrichtung der Kunststoffvorformlinge bewegbar.

Das Bezugszeichen 4 kennzeichnet eine Ventilanordnung wie einen Ventilblock, der bevorzugt eine Vielzahl von Ventilen aufweist, welche die Beaufschlagung der Kunststoffvorformlinge mit unterschiedlichen Druckniveaus steuern. Bevorzugt weist jede Umformungsstation einen derartigen Ventilblock auf.

Bevorzugt ist auch an jeder dieser Ventilanordnungen eine Drosseleinrichtung angeordnet, welche den Strömungsquerschnitt der den Kunststoffvorformlingen unter wenigstens einem Druck zugeführten Druckluft steuert. Wie oben erwähnt sind wenigstens einige dieser Ventileinrichtungen und bevorzugt alle dieser Ventileinrichtungen sterilisierbar.

Bei einem bevorzugten Verfahren werden die Kunststoffvorformlinge zunächst mit einem Vorblasdruck P1 beaufschlagt, anschließend mit mindestens einem Zwischenblasdruck Pi, der höher ist als der Vorblasdruck und schließlich mit einem Fertigblasdruck P2, der höher ist als der Zwischenblasdruck Pi1. Besonders bevorzugt werden die Kunststoffvorformlinge mit einem weiteren Zwischenblasdruck Pi2 beaufschlagt, der größer ist als der Druck Pi1 jedoch kleiner als der Druck P2.

Nach der Expansion der Kunststoffbehältnisse werden bevorzugt die Drücke bzw. die Druckluft wieder von dem Behältnis in die einzelnen Druckreservoirs zurückgeführt.

Bevorzugt weist die Vorrichtung daher eine Luftrecyceleinrichtung auf, welche dazu geeignet und bestimmt ist, Druckluft von den einzelnen Umformungsstationen in Druckluftreservoirs, insbesondere solche Druckluftreservoirs, welche ein geringeres Druckniveau aufnehmen, zurückzuführen.

Das Bezugszeichen 88 kennzeichnet eine Reckstange, die dazu dient, die Kunststoffvorformlinge in ihrer Längsrichtung zu dehnen. Bevorzugt weisen sämtliche Umformungsstationen derartige Blasformen 82 sowie Reckstangen 88 auf. Diese Reckstange ist bevorzugt Bestandteil einer mit 30 bezeichneten Reckeinrichtung. Die Reckstange ist (bevorzugt ebenfalls ausschließlich) in der Längsrichtung der Kunststoffvorformlinge 10 beweglich.

Bevorzugt liegt die Anzahl dieser Umformungsstationen 4 zwischen 2 und 100, bevorzugt zwischen 4 und 60, bevorzugt zwischen 6 und 40.

Die Kunststoffvorformlinge 10 werden über eine erste Transporteinrichtung 62 wie insbesondere aber nicht ausschließlich einen Transportstern der Vorrichtung zugeführt. Über eine zweite Transporteinrichtung 64 werden die Kunststoffbehältnisse 15 abtransportiert.

Das Bezugszeichen 7 kennzeichnet eine Druckbereitstellungseinrichtung wie etwa einen Kompressor oder auch einen Druckluftanschluss. Die Druckluft wird über eine Verbindungsleitung 72 zu einem Drehverteiler 74 gefördert und von diesem über eine weitere Leitung 76 an das erste Druckluftreservoir 32 angegeben, bei dem es sich hier um einen Ringkanal handelt.

Damit dient bevorzugt dieser Drehverteiler zu dem Zweck, Luft von einem stationären Teil der Vorrichtung in einen sich drehenden Teil der Vorrichtung zu führen.

Neben diesem dargestellten Ringkanal 32 sind bevorzugt noch weitere Ringkanäle vorgesehen, die in der in Fig. 1 gezeigten Darstellung jedoch durch den Ringkanal 32 verdeckt sind, beispielsweise darunterliegen. So ist bevorzugt je ein Druckreservoir zur Speicherung des Drucks P2 sowie der Zwischenblasdrücke Pi1 und Pi2 und des Drucks P1 vorhanden.

Das Bezugszeichen 33 kennzeichnet eine Verbindungsleitung, welche die Druckluft an eine Umformungsstation 20 bzw. deren Ventilblock 4 abgibt. Bevorzugt ist jeder der Ringkanäle über entsprechende Verbindungsleitungen mit sämtlichen Umformungsstationen verbunden. Diese Verbindungsleitung 33 ist bevorzugt in dem drehenden Teil der Vorrichtung angeordnet.

Das Bezugszeichen 18 kennzeichnet schematisch einen optionalen Reinraum, der hier bevorzugt ringförmig ausgebildet ist und den Transportpfad der Kunststoffvorformlinge 10 umgibt. Bevorzugt ist eine (geometrische) Drehachse bezüglich derer der Transportträger 22 drehbar ist, außerhalb des Reinraums 18 angeordnet. Bevorzugt ist der Reinraum mit einer Dichtungseinrichtung, welche bevorzugt wenigstens zwei Wasserschlösser aufweist, gegenüber der unsterilen Umgebung abgedichtet.

Weiterhin weist die Vorrichtung bevorzugt eine (in Fig. 1 nicht gezeigte) Deckeleinrichtung auf, welche den Reinraum 18 nach oben hin begrenzt. Diese Deckeleinrichtung ist dabei bevorzugt an wenigstens einer der Reckeinrichtungen 30 angeordnet.

Die Vorrichtung weist eine Vielzahl von Mess- und/oder Sensoreinrichtungen auf, welche zur Steuerung der Vorrichtung dienen. Das Bezugszeichen 14 kennzeichnet eine Druckmesseinrichtung, welche einen Luftdruck innerhalb des Druckluftreservoirs 32 misst. Bevorzugt weisen auch die übrigen Druckluftreservoirs entsprechende Druckmesseinrichtungen auf.

Das Bezugszeichen 16 kennzeichnet eine weitere Druckmesseinrichtung, welche einen Luftdruck insbesondere einen Behältnisinnendruck des zu expandierenden Kunststoffvorformlings misst. Bevorzugt ist jeder Umformungsstation eine solche Druckmesseinrichtung zugeordnet.

Bevorzugt weisen auch die Ventileinrichtungen selbst jeweils wenigstens eine und bevorzugt mehrere Sensoreinrichtungen wie Druckmesseinrichtungen auf. Daneben können die Ventileinrichtungen auch Sensoreinrichtungen zur Erfassung der Konzentration eines Sterilisationsmedium aufweisen.

Bevorzugt weist die Vorrichtung eine Sterilisationseinrichtung auf, welche dazu geeignet und bestimmt ist, die einzelnen Ventileinrichtungen mit einem fließfähigen Sterilisationsmedium zu beaufschlagen. Bevorzugt findet dabei diese Sterilisation in einem von einem Arbeitsbetrieb der Vorrichtung unterschiedlichen Sterilisationsbetrieb statt.

Das Bezugszeichen 19 kennzeichnet ebenfalls schematisch eine Durchflussmesseinrichtung, welche einen Durchfluss der Blasluft von einem Druckluftreservoir zu dem Ventilblock 4 einer Umformungsstation 20 bestimmt. Bevorzugt sind entsprechende Durchflussmesseinrichtungen jeweils zwischen einem Druckluftreservoir und allen Umformungsstationen angeordnet.

Auch zwischen den weiteren Druckluftreservoirs und den jeweiligen Umformungsstationen können weitere Durchflussmesseinrichtungen zugeordnet sein.

Weiterhin sind bevorzugt auch Positionserfassungseinrichtungen vorgesehen, welche Positionen der Reckstangen der einzelnen Umformungsstationen erfassen können.

Das Bezugszeichen 24 kennzeichnet eine Steuerungseinrichtung, welche die Vorrichtung 1 steuert und insbesondere regelt. Diese Steuerungseinrichtung ist dabei bevorzugt auch in der Lage, Arbeitsparameter der Vorrichtung und insbesondere Einstellungen der Drosseleinrichtung zu ändern.

So steuert die Steuerungseinrichtung insbesondere die einzelnen Ventileinrichtungen und damit die Beaufschlagung der Kunststoffvorformlinge mit den einzelnen Druckniveaus. Daneben steuert die Steuerungseinrichtung bevorzugt auch eine Bewegung der Reckstangen der einzelnen Umformungsstationen. Bevorzugt steuert die Steuerungseinrichtung auch Bewegungen der Beaufschlagungseinrichtungen d.h. der Blasdüsen. Wie erwähnt kann diese Steuerungseinrichtung bevorzugt auch die Einstellungen der einzelnen Drosseleinrichtungen einstellen.

Die Steuerungseinrichtung ist bevorzugt dazu geeignet, die Zeitpunkte, zu denen die Beaufschlagungseinrichtungen an die Kunststoffvorformlinge angelegt werden und/oder die Zeitpunkte, zu denen die Blasformeinrichtungen wieder von den Kunststoffvorformlingen abgehoben werden zu steuern und insbesondere auch, diese Zeitpunkte zu verändern.

Das Bezugszeichen 26 kennzeichnet eine Speichereinrichtung, in der insbesondere Messgrößen erfasst werden, insbesondere Druckwerte und Durchflusswerte, aber auch entsprechende Arbeitsparameter. Bevorzugt werden diese jeweiligen Werte mit einer zeitlichen Zuordnung gespeichert.

Bevorzugt können diese Werte kontinuierlich und insbesondere über lange Zeiträume eines Maschinenbetriebs gespeichert. Die Steuerungseinrichtung steuert bzw. regelt auch unter Berücksichtigung dieser aufgenommenen Messwerte die Vorrichtung.

Das Bezugszeichen 28 kennzeichnet grob schematisch eine Inspektionseinrichtung zum Inspizieren der gefertigten Behältnisse.

Das Bezugszeichen 25 kennzeichnet eine Anzeigeeinrichtung, welche zur Ausgabe von Informationen an einen Maschinenbediener dient. Mittels dieser Anzeigeeinrichtung können etwa gemessene Druck(verlaufs)kurven ausgegeben werden.

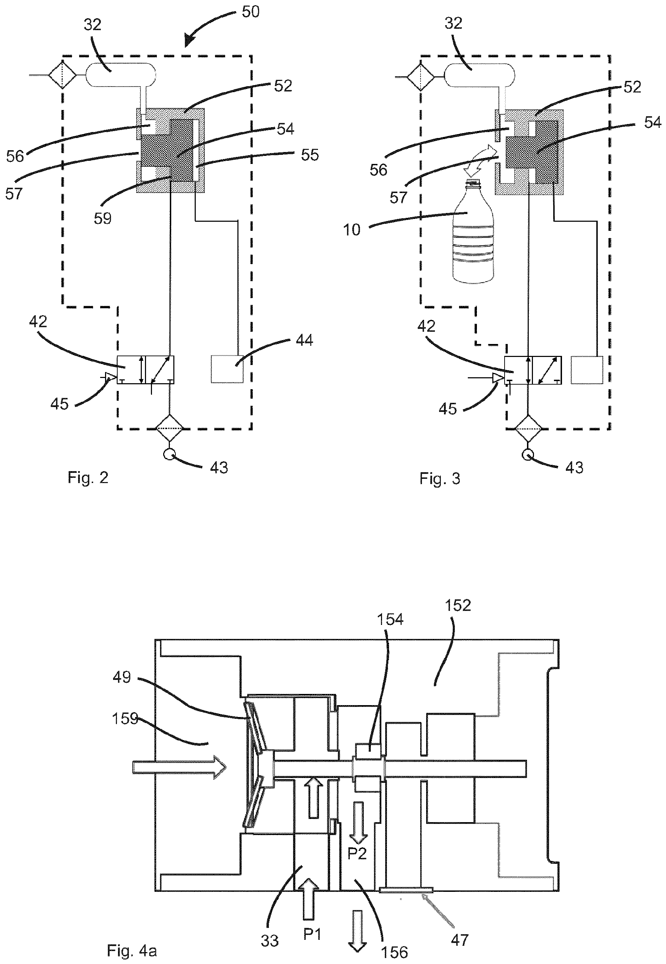

Die Fig. 2 und 3 zeigen eine weitere erfindungsgemäße Ventileinrichtung, wobei Fig. 2 eine geschlossene Position dieser Ventileinrichtung 50 und Fig. 3 eine geöffnete Position der Ventileinrichtung 50 zeigt.

Das Bezugszeichen 52 kennzeichnet ein Ventilgehäuse, innerhalb dessen ein Ventilkolben 54 linearbeweglich zwischen einer ersten Position (Fig. 2) und einer zweiten Position (Fig. 3) ist, um das Ventil zu schließen oder zu öffnen. Bevorzugt ist dieser Ventilkolben derart angeordnet, dass er in einem Sterilisationsbetrieb ständig von einem Sterilisationsmedium umspülbar ist.

Das Bezugszeichen 56 kennzeichnet einen Arbeitsraum der Ventileinrichtung 50, welcher zur Aufnahmen der Blasluft dient. Dieser Arbeitsraum ist durch ein Sterilisationsmedium sterilisierbar.

Das Bezugszeichen 32 kennzeichnet das Reservoir, welches die Blasluft zur Verfügung stellt. Das Bezugszeichen 57 kennzeichnet einen Ausgang des Ventilgehäuses 52 über welchen die Blasluft zu dem Behältnis 10 bzw. dem Kunststoffvorformling gelangt.

Das Bezugszeichen 59 bezieht sich auf einen Steuerraum, welcher mit einem Steuerfluid, insbesondere Steuerluft beaufschlagt werden kann, um den Ventilkolben gegenüber dem Gehäuse 52 in einer Richtung zu bewegen, hier von einer geschlossenen Stellung in die in Fig. 3 gezeigte geöffnete Stellung. Bei diesem Steuerfluid, welches ausgehend von einer Luftquelle 43 zugeführt wird, handelt es sich um sterile Steuerluft.

Das Bezugszeichen 42 kennzeichnet ein Steuerventil, welches die Zuführung der Steuerluft an den Steuerraum 59 steuert. Diesem Steuerventil 42 wird bevorzugt unsterile PilotLuft über einen Eingang 45 zugeführt. In dem Steuerventil 42 befindet sich die Sterilgrenze welche bevorzugt durch eine Membran gebildet wird. Auf diese Weise wird verhindert, dass die unsterile Pilotluft mit der sterilen Sterilluft in dem Arbeitsraum in Verbindung gelangen kann.

Die Sterilgrenze stellt dabei bevorzugt eine Ebene oder eine Trennung zwischen einem sterilien Bereich und einem nicht sterilen Bereich der Vorrichtung dar.

Bevorzugt ist Sterilgrenze durch ein Element ausgebildet. Bevorzugt ist die Sterilgrenze luftundurchlässig und verhindert, dass Keime von einem nicht aseptischen Bereich in einen aseptischen Bereich gelangen.

Alternativ oder zusätzlich kann die Sterilgrenze auch durch eine laminare Strömung vom sterilen in nicht sterilen Bereich erzeugt werden. Auch kann eine Sterilgrenze durch einen höheren Druck in einem sterilen Bereich gegenüber einem niedrigeren Druck in einem nicht sterilen Bereich erreicht werden.

Das Bezugszeichen 44 kennzeichnet eine Luftfederungseinrichtung, welche einem Luftfederungsraum 55 Luft zum Rückstellen des Ventilkolbens 54 zuführt.

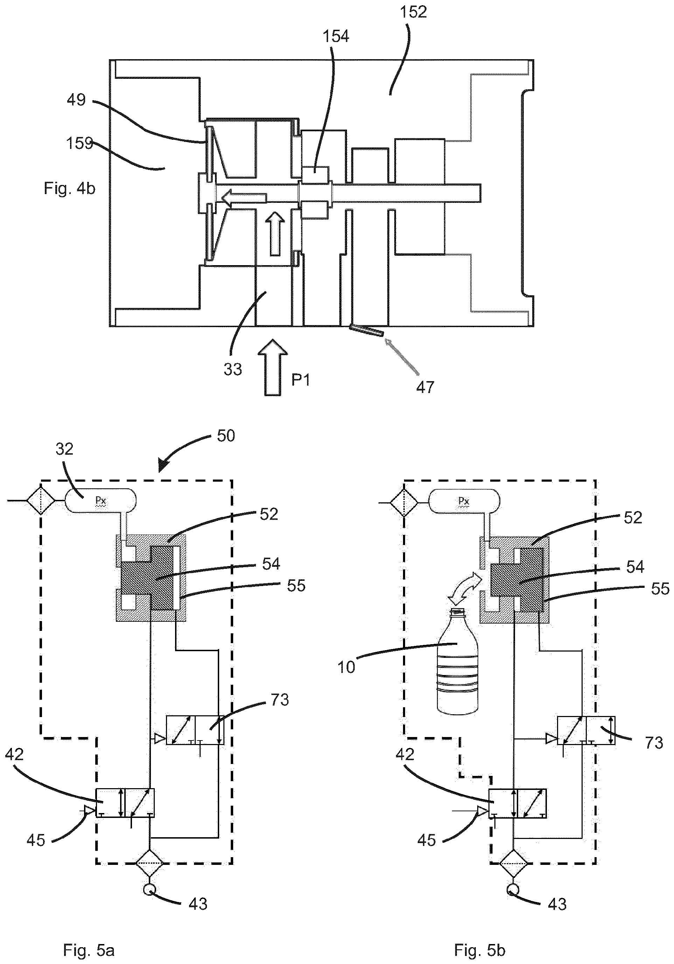

Die Fig. 4a und 4b zeigen detaillierte Ansicht des Steuerventils 42.

Bei diesem Steuerventil ist wiederum ein Ventilkolben 154 in einer geraden Richtung bewegbar. In der geöffneten Stellung gelangt sterile Steuerluft wie durch den Pfeil P1 gezeigt in das Steuerventil und wie durch den Pfeil P2 gezeigt von dem Steuerventil zu dem Steuerraum 159 in dem Ventilgehäuse 152.. Das Bezugszeichen 33 kennzeichnet entsprechend den Ventileingang und das Bezugszeichen 156 den Ventilausgang.

Das Bezugszeichen 159 kennzeichnet den Eingang des Steuerventils, in welchen die nicht sterile Pilotluft zur Bewegung des Ventilkolbens 154 des Steuerventils zugeführt wird. Das Bezugszeichen 49 kennzeichnet eine Membran, welche hier auch den Sterilraum bzw. den aseptischen Bereich der Ventileinrichtung von dem Steuerraum trennt.

Weiterhin ist der Druck der zugeführten Blasluft stets größer als der Druck der Steuerluft. Auf diese Weise kann auch bei einer Beschädigung der Membran 49 die Arbeitsluft nicht durch die Steuerluft verunreinigt werden.

Die Fig. 5a und 5b zeigen eine weitere Ausgestaltung einer erfindungsgemäßen Ventileinrichtung 50, wobei Fig 5a wiederum eine geschlossene Stellung und Fig. 5b eine geöffnete Stellung zeigt.

Der Unterschied zu der in Fig. 3a, b gezeigten Ausführungsform besteht darin, dass hier anstelle der dort gezeigten Luftfeder 44 ein weiteres pneumatisches Ventil 73 verwendet wird, um auch die Rückstellung des Ventilkolbens 54 zu steuern.

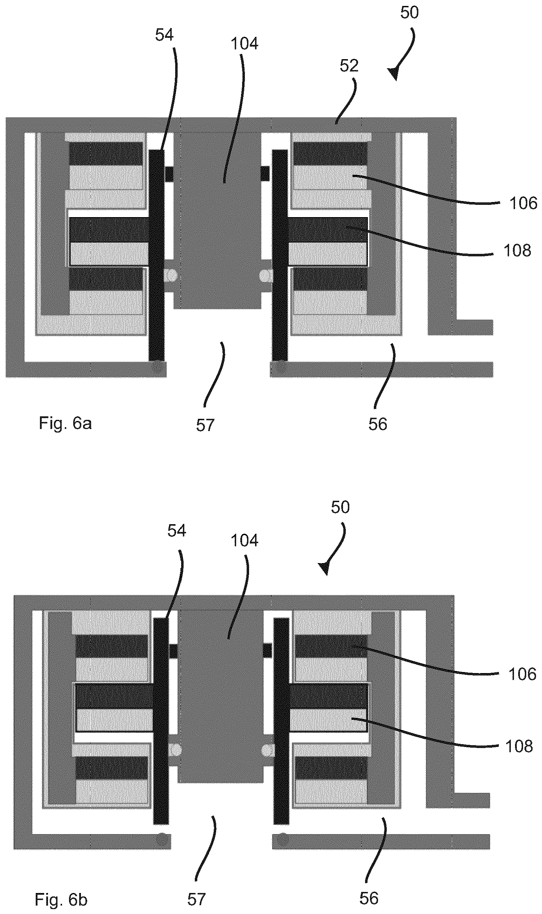

Die Fig. 6a, 6b zeigen eine weitere Ventileinrichtung 50, wobei Fig. 6a einen geschlossenen Zustand und Fig. 6b einen geöffneten Zustand der Ventileinrichtung zeigt. Das Bezugszeichen 54 bezieht sich wieder auf einen Ventilkolben, der zwischen zwei Positionen bewegbar ist.

Das Bezugszeichen 56 kennzeichnet den Arbeitsraum, welche mit dem gasförmigen Medium zum Expandieren der Kunststoffvorformlinge 10 beaufschlagbar ist.

Der Ventilkolben 54 wird durch eine Führungseinrichtung 104 geführt. Bei der in Fig. 6a, 6b gezeigten Ausführungsform ist eine Vielzahl von Permanentmagneten 106, 108 vorgesehen, die ebenfalls linear beweglich sind. Der Ventilkolben ist magnetisch mit diesen Permanentmagneten gekoppelt und folgt daher der Bewegung der Permanentmagneten.

Zwischen dem Ventilkolben und den Permanentmagneten ist weiterhin eine Wandung angeordnet, die einerseits als Sterilraumgrenze ausgebildet ist und insbesondere einen Durchtritt von Gasen verhindert und durch welche hindurch andererseits eine magnetische Anziehungskraft wirkt.

Die Bewegung der Permanentmagnete kann andererseits durch eine Vielzahl von Aktuatoreinrichtungen bewirkt werden, beispielsweise durch elektrische Antriebe oder etwa auch durch einen pneumatischen Antrieb.

Die Anmelderin behält sich vor sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Vorrichtung zum Umformen von Kunststoffvorformlingen (10) zu Kunststoffbehältnissen, mit einer Transporteinrichtung (2), welche die Kunststoffvorformlinge (10) entlang eines vorgegebenen Transportpfads transportiert, wobei die Transporteinrichtung (2) einen Transportträger (22) aufweist, an dem eine Vielzahl von Umformungsstationen (20) zum Umformen der Kunststoffvorformlinge (10) angeordnet ist, wobei diese Umformungsstationen jeweils Beaufschlagungseinrichtungen (84) aufweisen, welche die Kunststoffvorformlinge zu ihrer Expansion mit einem fließfähigen und insbesondere gasförmigen Medium beaufschlagen und wobei die Vorrichtung (1) wenigstens ein erstes Druckluftreservoir (32) aufweist, welches dazu geeignet und bestimmt ist, Druckluft unter einem ersten vorgegebenen Druck bereitzustellen und jede Umformungsstation (20) eine Ventilanordnung (4) mit einer Vielzahl von Ventileinrichtungen (50) aufweist, um die Kunststoffvorformlinge mit wenigstens zwei Drücken zu beaufschlagen,
**dadurch gekennzeichnet, dass**
wenigstens eine dieser Ventileinrichtungen (50) eine durch ein Sterilisationsmedium sterilisierbare pneumatisch gesteuerte Ventileinrichtung oder eine durch ein Sterilisationsmedium sterilisierbare magnetisch und/oder durch Magnetkraft betätigbare Ventileinrichtung ist.

2. Vorrichtung (1) nach Anspruch 1,
**dadurch gekennzeichnet, dass**
die Ventileinrichtung balglos ausgeführt ist.

3. Vorrichtung (1) nach wenigstens einem der vorangegangenen Ansprüche,
**dadurch gekennzeichnet, dass**
die Ventileinrichtung einen Arbeitsraum aufweist, welcher dazu geeignet und bestimmt ist, wenigstens zeitweise das zur Expansion der Kunststoffvorformlinge (10) verwendete gasförmige Medium aufzunehmen, sowie einen Steuerraum zum Aufnehmen der zum Steuern der Ventileinrichtung verwendeten Luft, wobei sowohl der Arbeitsraum als auch der Steuerraum von dem Sterilisationsmedium sterilisierbar sind.

4. Vorrichtung (1) nach wenigstens einem der vorangegangenen Ansprüche,
**dadurch gekennzeichnet, dass**
die Vorrichtung (1) einen Reinraum (18) aufweist, innerhalb dessen die umzuformenden Kunststoffvorformlinge transportiert werden.

5. Vorrichtung nach wenigstens einem der vorangegangenen Ansprüche,
**dadurch gekennzeichnet, dass**
das Steuerventil 42 ein 3/2 Wegeventil oder ein 5/2 Wegeventil ist.

6. Vorrichtung nach wenigstens einem der vorangegangenen Ansprüche,
**dadurch gekennzeichnet, dass**
die Ventileinrichtung (50) mit einer Rückstelleinrichtung (44,73) zur Rückstellung eines Ventilkolbens der Ventileinrichtung ausgestattet ist, wobei diese Rückstelleinrichtung eine Luftfederungseinrichtung (44) oder eine weitere Ventileinrichtung (73) aufweist.

7. Vorrichtung nach wenigstens einem der vorangegangenen Ansprüche,
**dadurch gekennzeichnet, dass**
ein Steuerdruck zum Ansteuern der Ventileinrichtung niedriger ist als ein Druck des gasförmigen Mediums zum Expandieren der Kunststoffvorformlinge.

8. Vorrichtung nach wenigstens einem der vorangegangenen Ansprüche,
**dadurch gekennzeichnet, dass**
die Vorrichtung ein zweites Druckluftreservoir aufweist, welches dazu geeignet und bestimmt ist, Druckluft unter einem zweiten vorgegebenen Druck (pi) bereitzustellen, wobei dieser zweite Druck höher ist als der erste Druck (p1).

9. Vorrichtung nach wenigstens einem der vorangegangenen Ansprüche,
**dadurch gekennzeichnet, dass**
ein magnetischer Aktuator der Ventileinrichtung durch magnetkraftdurchlässige Wandung von einem Kolben der Ventileinrichtung getrennt ist.

10. Vorrichtung nach wenigstens einem der vorangegangenen Ansprüche,
**dadurch gekennzeichnet, dass**
die Ventileinrichtung eine Permanentmagnetanordnung (106, 108) und insbesondere eine bewegbare Permanentmagnetanordnung aufweist.

11. Vorrichtung nach wenigstens einem der vorangegangenen Ansprüche,
**dadurch gekennzeichnet, dass**
die Ventileinrichtung (50) ein Steuerventil (42) und ein Hauptventil aufweist, wobei eine Sterilgrenze in dem Steuerventil angeordnet ist und/oder sich ein Hauptkolben der Ventileinrichtung, der die Blasluft steuert stets innerhalb eines Raums befindet, der mit aseptischer Luft umspült werden kann.

12. Vorrichtung nach wenigstens einem der vorangegangenen Ansprüche,
**dadurch gekennzeichnet, dass**
dem Steuerventil ein Pilotventil vorgeschaltet ist, wobei alle Ventile aseptisch ausgeführt sind.

13. Vorrichtung (1) nach wenigstens einem der vorangegangenen Ansprüche,
**dadurch gekennzeichnet, dass**
die Vorrichtung (1) sowohl in einem Arbeitsbetrieb betreibbar ist, in welchem die Kunststoffvorformlinge (10) umgeformt werden als auch in einem Sterilisationsbetrieb, in welchem die Vorrichtung (1) und/oder die Ventileinrichtung sterilisiert wird.

14. Verfahren zum Umformen von Kunststoffvorformlingen (10) zu Kunststoffbehältnissen, wobei eine Transporteinrichtung (2) die Kunststoffvorformlinge (10) entlang eines vorgegebenen Transportpfads (P) transportiert, wobei die Transporteinrichtung (2) einen Transportträger (22) aufweist, an dem eine Vielzahl von Umformungsstationen (20) zum Umformen der Kunststoffvorformlinge (10) angeordnet ist, wobei diese Umformungsstationen jeweils Beaufschlagungseinrichtungen (84) aufweisen, welche die Kunststoffvorformlinge (10) zu ihrer Expansion mit einem fließfähigen und insbesondere gasförmigen Medium beaufschlagen und wobei die Vorrichtung (1) wenigstens ein erstes Druckluftreservoir (32) aufweist, welches Druckluft unter einem ersten vorgegebenen Druck bereitstellt und jede Umformungsstation (20) eine Ventilanordnung (4) mit einer Vielzahl von Ventileinrichtungen (50) aufweist, um die Kunststoffvorformlinge mit wenigstens zwei Drücken zu beaufschlagen,
**dadurch gekennzeichnet, dass**
wenigstens eine dieser Ventileinrichtungen (50) eine durch ein Sterilisationsmedium sterilisierbare pneumatisch gesteuerte Ventileinrichtung oder eine durch ein Sterilisationsmedium sterilisierbare magnetisch gesteuerte Ventileinrichtung und/oder eine durch Magnetkraft betätigbare Ventileinrichtung ist.

15. Verfahren nach dem vorangegangenen Anspruch,
**dadurch gekennzeichnet, dass**
in einem Arbeitsraum der Ventileinrichtung, der zur Aufnahme des die Kunststoffvorformlinge beaufschlagenden gasförmigen Mediums dient, während eines Arbeitsbetriebs ein höherer Druck herrscht als in einem Steuerraum, der zur Aufnahme eines Steuerfluids dient, welches einen im Arbeitsraum angeordneten Ventilkolben der Ventileinrichtung bewegt.
